# EUROPEAN PATENT APPLICATION

(11) **EP 4 749 504 A1**
(43) Date of publication of application: **27.05.2026**
(21) Application number: 24220618.3
(22) Date of filing: 17.12.2024
(51) Int. Cl.: G06F 30/13, G01N 33/46, G06F 30/20

(54) **SMART WOOD PERFORMANCE AND PREDICTION VISUALIZATION FOR STRUCTURAL HEALTH MONITORING**

(30) Priority: 25.11.2024 BE 202405825
(71) Applicant: Tallinna Tehnikaülikool, 19086 Tallinn (EE)
(72) Inventor: Kuusik, Alar, 75201 Raasiku vald (EE)
(74) Representative: Bringer IP

(57) **Abstract**

Smart wood performance prediction and visualization for structural health monitoring (SHM) includes loading an SHM model for a generic timber structure and establishing communications with different SHM sensors fixed to different dimensional lumber segments of an actual timber structure. Wood performance data is then retrieved from each SHM sensor in connection with a contemporaneous period of acquisition and a corresponding dimensional lumber segment. As such, the wood performance data is compared to a predicted performance stored in the SHM model in respect to the contemporaneous period. Finally, an anomalous value of the wood performance data that deviates from an expected value of the predicted performance at the contemporaneous period is identified, and a visualization is generated which indicates the anomalous value and identifies both an associated dimensional lumber segment and also a location of the associated dimensional lumber segment in the actual timber structure.

## Description

### BACKGROUND OF THE INVENTION

### Field of the Invention

The present invention relates to the technical field of structural health monitoring (SHM) of a timber structure.

### Description of the Related Art

The reduction of carbon footprint in building construction requires the extensive use of wooden materials instead of steel and polymer materials. The use of wooden materials, however, is not without consequence. Specifically, wood as a material is susceptible to degradation over time, in consequence of moisture and temperature impacts, and as such, wood exhibits a high variability in its physical and mechanical properties. As noted by Olaf Schmidt in "Indoor wood-decay basidiomycetes: damage, causal fungi, physiology, identification and characterization, prevention and control." Mycological Progress 6, no. 4 (2007): 261-279, according to the standard EN 335:2013, the development of fungi is expected to begin from a wood moisture content of ≥ 20 %.

Further, as noted by Hannu A. Viitanen in "Modelling the Time Factor in the Development of Brown Rot Decay in Pine and Spruce Sapwood - The Effect of Critical Humidity and Temperature Conditions", Holzforschung 51, at 99-106 (1997) wood-rotting fungi begins to grow from ≥ 25 % of moisture content. The development is more likely at air temperatures +5..+10°C and high air relative humidity levels and slows down on temperatures below 0°C and above 50°C. Indeed, as stated in Hradil, Petr, and Stefania Fortino. "Integrated approach to predict deterioration of mechanical properties of decaying wood" in 13th World Conference on Timber Engineering, WCTE 2023, even small mass loss of 8% due to decay drastically (50%) decreases wood strength in bending. Wood when structurally dimensioned in the form of timber exhibits high variability not only between but also within elements, mainly due to non-homogeneities, such as knots and deviations in grain. These non-homogeneities affect the performance of wood as a construction material.

Of particular note, water leakages and construction errors cause development of wood-decay fungi, such as Antrodia spp., Gloeophyllum, Coniophora, Donkioporia, Serpula lacrymans. This decay has been found to be a primary cause of deteriorating of wooden structures. To wit, the natural deterioration of wooden material, especially cladding, is due to environmental impact including temperature variations and rain). The deterioration speed therefore is subject of climate conditions at specific geographic location. As such, any type of water damage can facilitate growth of mold in as soon as twenty-four to forty-eight hours. For instance, it has further been found that one out of six houses in Europe have mold. In the United States, mold has led to a twenty to thirty-seven percent drop in home resale value, not in small part owing to the correlation between fungi and severe health issues resulting from mold illness.

SHM thus has proven to be an important emerging technology intended to mitigate the impact of the degredation of structurally dimensioned lumber in a timber structure. SHM refers to the instrumentation of structurally dimensioned lumber in order to measure strain, moisture and temperature, among other metrics, in respect to the lumber. With SHM, temperature and moisture impacts upon structurally dimensioned lumber can be monitored in order to determine when an undesireable degree of wood degredation has occurred. Conseqeuntly, given SHM, it has been recognized that the early detection of abnormal moisture level in wooden structures is crucial for preserving long lifetime of wooden constructions and a healthy indoor climate. For example, use of water sprinkler fire extinguishing systems is problematic in wooden constructions due to fact that water caused damages can exceed fire caused damages. SHM systems can be used for localizing wet areas after the fire distinguishing operations to minimize the damages caused by extinguishing water. Temperature or gas detection SHM systems can be used for localizing a fire and observing burning process evolving in wood-based constructions.

### BRIEF SUMMARY OF THE INVENTION

Embodiments of the present invention address technical deficiencies of the art in respect to SHM for a timber structure. To that end, embodiments of the present invention provide for a novel and non-obvious method for smart wood performance prediction and visualization for SHM. Embodiments of the present invention also provide for a novel and non-obvious computing device adapted to perform the foregoing method. Finally, embodiments of the present invention provide for a novel and non-obvious data processing system incorporating the foregoing device in order to perform the foregoing method.

In one embodiment of the invention, smart wood performance prediction and visualization for SHM includes loading in memory of a host computing platform, an SHM model for a generic timber structure. Then, a communicative coupling is established between the host computing platform and different SHM sensors fixed to different dimensional lumber segments of an actual timber structure. Wood performance data from each SHM sensor is then loaded into memory of the host computing platform in connection with a contemporaneous period of acquisition and a corresponding dimensional lumber segment.

The wood performance data is then compared to a predicted performance stored in the SHM model in respect to the contemporaneous period. Finally, an anomalous value of the wood performance data that deviates from an expected value of the predicted performance at the contemporaneous period is identified in association with a subject dimensional lumber segment. As such, a visualization is generated in a display of the host computing platform indicating the anomalous value or an anomalous trend of values over time, and identifying both the subject dimensional lumber segment and also a location of the dimensional lumber segment in the actual timber structure.

Different aspects of the embodiment can include the following aspects alone or in various combinations with one another:
- The SHM model includes an expected performance of different ones of the dimensional lumber segments when arranged at different locations of the generic timber structure during different times of a year.
- The SHM sensors measure moisture at least one of material internal moisture, material internal conductivity, material strain, material surface temperature, construction element tilt (angle) sensor, air CO2 and CO concentration, volatile organic compounds (VOC) and volatile sulfur compounds (VSC) in the air, air moisture and temperature, ultraviolet and visible light exposure, crack sound detector.
- The visualization is a heat map of the actual timber structure differentiating locations at which ones of the dimensional lumber segments have exhibited nominal performance and others of the dimensional lumber segments have exhibited anomalous performance according to the loaded wood performance data.
- The communicative coupling is a real-time wireless communications link between the host computing platform and one or several of the SHM sensors.
- The communicative coupling is an uploaded table of the wood performance data collected from each of the SHM sensors.
- The SHM model reflects an average of wood historical performance data of different dimensional wood segments in different actual time structures collected at different periods of time.

In another embodiment of the invention, a data processing system is adapted for smart wood performance prediction and visualization for SHM. The system includes a host computing platform of one or more computers, each with memory and one or processing units including one or more processing cores. The system also includes a structural health monitoring module. The module in turn includes computer program instructions which are enabled while executing in the memory of at least one of the processing units of the host computing platform to perform smart wood performance prediction and visualization for SHM.

To that end, smart wood performance prediction and visualization for SHM includes loading into the memory of the host computing platform, an SHM model for a generic timber structure and establishing a communicative coupling between the host computing platform and different SHM sensors fixed to different dimensional lumber segments of an actual timber structure. Smart wood performance prediction and visualization for SHM further includes loading wood performance data from each of the SHM sensors into the memory of the host computing platform in connection with a contemporaneous period of acquisition and a corresponding one of the dimensional lumber segments and comparing the wood performance data to a predicted performance stored in the SHM model in respect to the contemporaneous period.

Finally, smart wood performance prediction and visualization for SHM includes identifying in association with a subject one of the dimensional lumber segments, an anomalous value of the wood performance data that deviates from an expected value of the predicted performance at the contemporaneous period, and generating a visualization in a display of the host computing platform indicating the anomalous value and identifying both the subject one of the dimensional lumber segments and also a location of the subject one of the dimensional lumber segments in the actual timber structure. In this way, the technical deficiencies of the wood construction are overcome owing to the windowed SHM model and its comparison to contemporaneously sourced SHM data from different segments of dimensionally structured lumber in a timber structure.

Additional aspects of the invention will be set forth in part in the description which follows, and in part will be obvious from the description, or may be learned by practice of the invention. The aspects of the invention will be realized and attained by means of the elements and combinations particularly pointed out in the appended claims. It is to be understood that both the foregoing general description and the following detailed description are exemplary and explanatory only and are not restrictive of the invention, as claimed.

### BRIEF DESCRIPTION OF THE SEVERAL VIEWS OF THE DRAWINGS

The accompanying drawings, which are incorporated in and constitute part of this specification, illustrate embodiments of the invention and together with the description, serve to explain the principles of the invention. The embodiments illustrated herein are presently preferred, it being understood, however, that the invention is not limited to the precise arrangements and instrumentalities shown, wherein:
Figure 1 is a pictorial illustration reflecting different aspects of a process of smart wood performance prediction and visualization for SHM;
Figure 2 is a block diagram depicting a data processing system adapted to perform one of the aspects of the process of Figure 1; and,
Figure 3 is a flow chart illustrating one of the aspects of the process illustrated in Figure 1.

### DETAILED DESCRIPTION OF THE INVENTION

Embodiments of the invention provide for smart wood performance prediction and visualization for SHM. In accordance with an embodiment of the invention, an SHM model is trained to reflect the predicted state of health for different lumber segments at different times of the year under different environmental conditions. The model can be trained, for instance, by accumulating state of health data for timber under a variety of conditions at different locations in different structure after different periods of time from the fabrication of the lumber segments. Thereafter, different dimensional lumber segments can be instrumented with respectively different SHM sensors and used in the construction of a timber structure. Subsequently, SHM values for each of the instrumented segments collected at a specific period of time are compared to the SHM model in order to identify threshold differences. A visualization of the timber structure, for example a heat map, is then generated with portions of the visualization corresponding to dimensional lumber of anomalous SHM values being rendered distinctively from those of nominal SHM values.

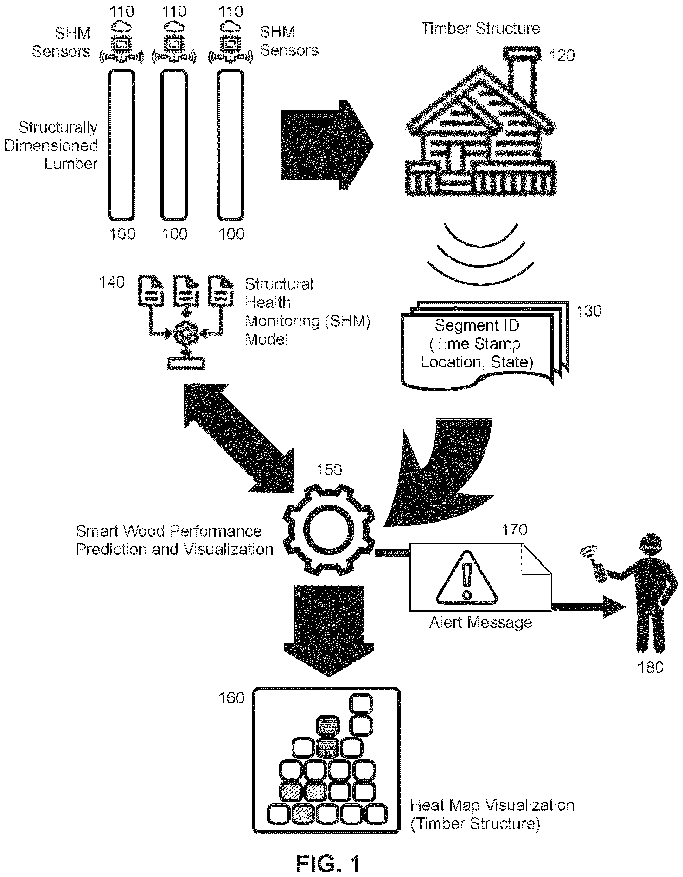

In illustration of one aspect of the embodiment, Figure 1 pictorially shows a process of smart wood performance prediction and visualization for SHM. As shown in Figure 1, different segments of structurally dimensioned lumber 100 are instrumented with respectively different SHM sensors 110 adapted to sense structure health aspects of the structurally dimensioned lumber 100 such as material moisture, conductivity, volatile organic and sulfur compounds in air, air moisture and temperature, strain, and the like. The segments of the structurally dimensioned lumber 100 are then included in the construction of a timber structure 120 from which, different SHM values 130 are reported from time to time or on demand, including a time stamp when the SHM values 130 are acquired, a location of an associated one of the segments of the structurally dimensioned lumber 100.

Smart wood performance prediction and visualization logic 150 compares the SHM values 130 of the timber structure 120 to an SHM model 140. As one example, a graphical curve can be defined for the SHM values 130 as acquired and the graphical curve is then compared to a counterpart graphical curve for the SHM values of the SHM model 140 in order to detect a threshold variance between the curves. Of note, in the comparison, the SHM values 130 are compared to SHM values of the SHM model 140 in respect to a common period of time when the SHM values 130 had been acquired as indicated by the corresponding time stamps. In this regard, SHM values 130 collected in the Winter period of time, or during evening hours, are compared to values in the SHM model 140 associated with the Winter period or the evening hours to the exclusion of other values in the SHM model 140.

The smart wood performance and visualization logic 150 identifies ones of the SHM values 130 which are threshold divergent from the values of the SHM model 140 and generates a visualization 160 such as a heat map which indicates those location of the timber structure 120 with segments of the structurally dimensioned lumber 100 reporting anomalous ones of the SHM values 130. As well, the smart wood performance and visualization logic 150 generates an alert message 170 including the location of the timber structure 120 with segments of the structurally dimensioned lumber 100 reporting anomalous ones of the SHM values 130. Thereafter, the smart wood performance and visualization logic 150 transmits the alert message 170 to a communications device of a designated operator 180.

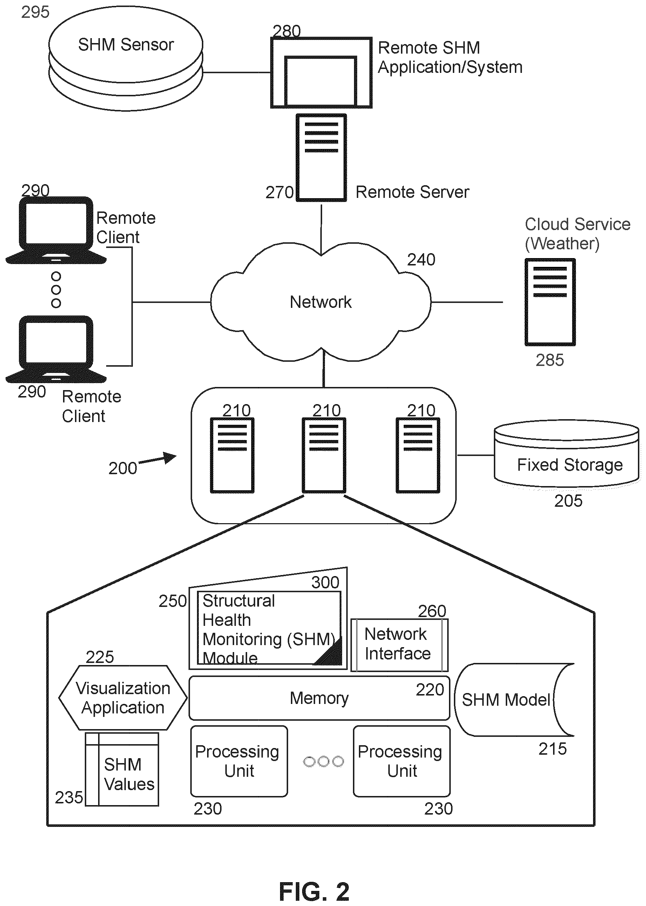

Aspects of the process described in connection with Figure 1 can be implemented within a data processing system. In further illustration, Figure 2 schematically shows a data processing system adapted to perform smart wood performance prediction and visualization for SHM. In the data processing system illustrated in Figure 1, a host computing platform 200 is provided. The host computing platform 200 includes one or more computers 210, each with memory 220 and one or more processing units 230, fixed storage 205 and a network interface 260. The computers 210 of the host computing platform (only a single computer shown for the purpose of illustrative simplicity) can be co-located within one another and in communication with one another over a local area network, or over a data communications bus.

A remote SHM application system 280 aggregating sensor measurements from a set of SHM sensors 295 executes in a remote server 270 communicatively coupled to the host computing platform 200 over a data communications network 240. The remote SHM application system 280 collects SHM values wireless from different SHM sensors in a timber structure and provides access to those SHM values to logic executing in the host computing platform 200. In this regard, the SHM values can be rendered accessible to the host computing platform 200 either directly through a real-time communicative coupling are as an uploaded table of SHM values collected from each of the SHM sensors. Different remote clients 290 communicatively access the host computing platform 200 from over the data communications network 240 and report observed SHM values for segments of dimensionally structured lumber at different locations in different timber structures during different periods of time.

Notably, a computing device 250 including a non-transitory computer readable storage medium can be included with the data processing system 200 and accessed by the processing units 230 of one or more of the computers 210. The computing device stores 250 thereon or retains therein a program module 300 that includes computer program instructions which when executed by one or more of the processing units 230, performs a programmatically executable process for smart wood performance prediction and visualization for SHM. Specifically, the program instructions during execution collect SHM values from the remote SHM application system 280 into an SHM values table 235 for an associated timber structure at a specified period of time. Optionally, the program instructions further collect meteorological information for the environment of the associated timber structure from a remotely coupled cloud service 285.

The program instructions then compare those collected values to values in an SHM model 215 in the memory 220 with the associated period of time. The program instructions then detect ones of the SHM values in the table 235 which are threshold deviant from counterpart values in the SHM model 215. Finally, the program instructions direct a visualization application 225 executing in the host computing platform 200 to generate a visualization highlighting the deviant locations of the timber structure relative to the nominal locations of the timber structure.

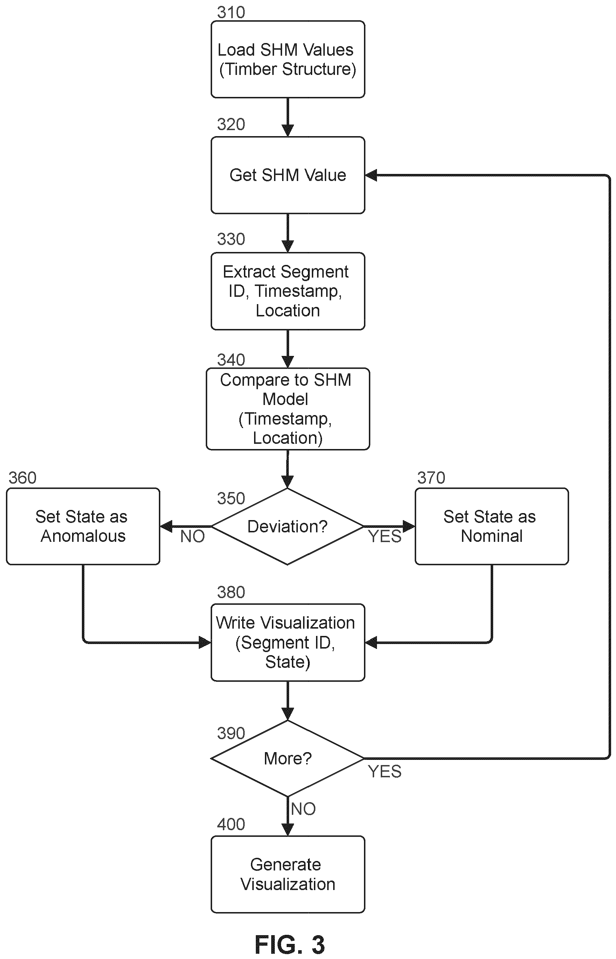

In further illustration of an exemplary operation of the module, Figure 3 is a flow chart illustrating one of the aspects of the process of Figure 1. Beginning in block 310, SHM values are loaded for an actual timber structure. In block 320, a first of the SHM values is retrieved and in block 330, a segment identifier is determined for the SHM value indicating the segment of dimensionally structured lumber from which the SHM value had been measured. As well, a timestamp when the SHM value had been acquired and a location within the actual timber structure of the segment is extracted from the SHM value. Then, in block 340, the SHM value is compared to a corresponding value for the timestamp and location.

In decision block 350, if a threshold deviation is determined between the SHM value that may consist of discrete sensor readings or fragments of their time series patterns measured for the segment of the dimensionally structured timber and the value of the SHM model, in block 360, a state of the SHM value is set as anomalous. But otherwise in block 370 the state of the SHM value is set as nominal. Thereafter, in block 380 a visualization entry is written for the SHM value in connection with the segment identifier and the state. In decision block 390, if further SHM values remain to be evaluated, the process returns to block 320 with the retrieval of a next SHM value for the segments of the actual timber structure. But, in decision block 390 if no further SHM values for the actual timber structure remain to be evaluated, in block 400 a visualization is generated for the actual timber structure showing those locations of the actual timber structure at which segments of the dimensionally structured lumber with SHM values that are anomalous are visually emphasized as in the case of a heatmap.

Of import, the foregoing flowchart and block diagram referred to herein illustrate the architecture, functionality, and operation of possible implementations of systems, methods, and computing devices according to various embodiments of the present invention. In this regard, each block in the flowchart or block diagrams may represent a module, segment, or portion of instructions, which includes one or more executable instructions for implementing the specified logical function or functions. In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

More specifically, the present invention may be embodied as a programmatically executable process. As well, the present invention may be embodied within a computing device upon which programmatic instructions are stored and from which the programmatic instructions are enabled to be loaded into memory of a data processing system and executed therefrom in order to perform the foregoing programmatically executable process. Even further, the present invention may be embodied within a data processing system adapted to load the programmatic instructions from a computing device and to then execute the programmatic instructions in order to perform the foregoing programmatically executable process.

To that end, the computing device is a non-transitory computer readable storage medium or media retaining therein or storing thereon computer readable program instructions. These instructions, when executed from memory by one or more processing units of a data processing system, cause the processing units to perform different programmatic processes exemplary of different aspects of the programmatically executable process. In this regard, the processing units each include an instruction execution device such as a central processing unit or "CPU" of a computer. One or more computers may be included within the data processing system. Of note, while the CPU can be a single core CPU, it will be understood that multiple CPU cores can operate within the CPU and in either instance, the instructions are directly loaded from memory into one or more of the cores of one or more of the CPUs for execution.

Aside from the direct loading of the instructions from memory for execution by one or more cores of a CPU or multiple CPUs, the computer readable program instructions described herein alternatively can be retrieved from over a computer communications network into the memory of a computer of the data processing system for execution therein. As well, only a portion of the program instructions may be retrieved into the memory from over the computer communications network, while other portions may be loaded from persistent storage of the computer. Even further, only a portion of the program instructions may execute by one or more processing cores of one or more CPUs of one of the computers of the data processing system, while other portions may cooperatively execute within a different computer of the data processing system that is either co-located with the computer or positioned remotely from the computer over the computer communications network with results of the computing by both computers shared therebetween.

The corresponding structures, materials, acts, and equivalents of all means or step plus function elements in the claims below are intended to include any structure, material, or act for performing the function in combination with other claimed elements as specifically claimed. The description of the present invention has been presented for purposes of illustration and description but is not intended to be exhaustive or limited to the invention in the form disclosed. Many modifications and variations will be apparent to those of ordinary skill in the art without departing from the scope and spirit of the invention. The embodiment was chosen and described in order to best explain the principles of the invention and the practical application, and to enable others of ordinary skill in the art to understand the invention for various embodiments with various modifications as are suited to the particular use contemplated.

Having thus described the invention of the present application in detail and by reference to embodiments thereof, it will be apparent that modifications and variations are possible without departing from the scope of the invention defined in the appended claims as follows:

## Claims

1. A method for smart wood performance prediction and visualization for structural health monitoring (SHM), comprising:
loading in memory of a host computing platform, an SHM model for a generic timber structure;
establishing a communicative coupling between the host computing platform and different SHM sensors fixed to different dimensional lumber segments of an actual timber structure;
loading wood performance data from each of the SHM sensors into the memory of the host computing platform in connection with a contemporaneous period of acquisition and a corresponding one of the dimensional lumber segments;
comparing the wood performance data to a predicted performance stored in the SHM model in respect to the contemporaneous period;
identifying in association with a subject one of the dimensional lumber segments, an anomalous value of the wood performance data that deviates from an expected value of the predicted performance at the contemporaneous period; and,
generating a visualization in a display of the host computing platform indicating the anomalous value or trend and identifying both the subject one of the dimensional lumber segments and also a location of the subject one of the dimensional lumber segments in the actual timber structure.

2. The method of claim 1, wherein the SHM model includes an expected performance of different ones of the dimensional lumber segments when arranged at different locations of the generic timber structure during a period selected from the group consisting of different times of a day and different times of a year.

3. The method of claim 1, wherein the SHM sensors measure moisture at least one of material internal moisture, internal conductivity, strain, temperature, carbon monoxide and carbon dioxide concentration in air, concentration of volatile organic compounds in air, concentration of volatile sulfur compounds in air, air moisture and temperature, ultraviolet light exposure, and cracking sound emanating from cracks in the subject one of the dimensional lumber segments.

4. The method of claim 1, wherein the visualization is a heat map of the actual timber structure differentiating locations at which ones fo the dimensional lumber segments have exhibited nominal performance and others of the dimensional lumber segments have exibited anomalous performance according to the loaded wood performance data.

5. The method of claim 1, wherein the communicative coupling is a real-time wireless communications link between the host computing platform and one of the SHM sensors.

6. The method of claim 1, wherein the communicative coupling is an uploaded table of the wood performance data collected from each of the SHM sensors.

7. The method of claim 1, wherein the SHM model reflects an average of wood historical performance data of different dimensional wood segments in different actual time structures collected at different periods of time.

8. A data processing system adapted for smart wood performance prediction and visualization for structural health monitoring (SHM), the system comprising:
a host computing platform comprising one or more computers, each with memory and one or processing units including one or more processing cores; and,
a structural health monitoring module comprising computer program instructions enabled while executing in the memory of at least one of the processing units of the host computing platform to perform:
loading into the memory of the host computing platform, an SHM model for a generic timber structure;
establishing a communicative coupling between the host computing platform and different SHM sensors fixed to different dimensional lumber segments of an actual timber structure;
loading wood performance data from each of the SHM sensors into the memory of the host computing platform in connection with a contemporaneous period of acquisition and a corresponding one of the dimensional lumber segments;
comparing the wood performance data to a predicted performance stored in the SHM model in respect to the contemporaneous period;
identifying in association with a subject one of the dimensional lumber segments, an anomalous value of the wood performance data that deviates from an expected value of the predicted performance at the contemporaneous period; and,
generating a visualization in a display of the host computing platform indicating the anomalous value and identifying both the subject one of the dimensional lumber segments and also a location of the subject one of the dimensional lumber segments in the actual timber structure.

9. The system of claim 8, wherein the SHM model includes an expected performance of different ones of the dimensional lumber segments when arranged at different locations of the generic timber structure during different times of a year.

10. The system of claim 8, wherein the SHM sensors measure moisture at least one of material internal moisture, internal conductivity, strain, temperature, carbon monoxide and carbon dioxide concentration in air, concentration of volatile organic and sulfur compounds in air, air moisture and temperature, ultraviolet light exposure, and cracking sound emanating from cracks in the subject one of the dimensional lumber segments.

11. The system of claim 8, wherein the visualization is a heat map of the actual timber structure differentiating locations at which ones of the dimensional lumber segments have exhibited nominal performance and others of the dimensional lumber segments have exibited anomalous performance according to the loaded wood performance data.

12. The system of claim 8, wherein the visualization is a heat map of the actual timber structure differentiating locations at which ones of the dimensional lumber segments have exhibited nominal temperature and gas concentration relative to others of the dimensional lumber segments which have exhibited anomalous temperature and gas concentration in order to assess a distribution and progression of fire within the actual timber structure.

13. The method of claim 8, wherein the communicative coupling is an uploaded table of the wood performance data collected from each of the SHM sensors.

14. The system of claim 8, wherein the SHM model reflects an average of wood historical performance data of different dimensional wood segments in different actual time structures collected at different periods of time.

15. A computing device comprising a non-transitory computer readable storage medium having program instructions stored therein, the instructions being executable by at least one processing core of a processing unit to cause the processing unit to perform smart wood performance prediction and visualization for structural health monitoring by:
loading in memory of a host computing platform, an SHM model for a generic timber structure;
establishing a communicative coupling between the host computing platform and different SHM sensors fixed to different dimensional lumber segments of an actual timber structure;
loading wood performance data from each of the SHM sensors into the memory of the host computing platform in connection with a contemporaneous period of acquisition and a corresponding one of the dimensional lumber segments;
comparing the wood performance data to a predicted performance stored in the SHM model in respect to the contemporaneous period;
identifying in association with a subject one of the dimensional lumber segments, an anomalous value of the wood performance data that deviates from an expected value of the predicted performance at the contemporaneous period; and,
generating a visualization in a display of the host computing platform indicating the anomalous value and identifying both the subject one of the dimensional lumber segments and also a location of the subject one of the dimensional lumber segments in the actual timber structure.
